# EUROPEAN PATENT SPECIFICATION

(11) **EP 1 870 045 B1**
(45) Date of publication and mention of the grant of the patent: **10.06.2009**
(21) Application number: 07252530.6
(22) Date of filing: 22.06.2007
(51) Int. Cl.: A61B 17/32, A61B 17/22

(54) **Ultrasonic probe deflection sensor**
Ultraschallsonde mit Ablenkungssensor
Capteur à défection de sonde à ultrasons

(30) Priority: 22.06.2006 US 473097
(43) Date of publication of application: 26.12.2007
(73) Proprietor: Tyco Healthcare Group LP, Norwalk, Connecticut 06856 (US)
(72) Inventor: Blier, Ken, Meriden, CT 06450 (US)
(74) Representative: Alcock, David

(56) References cited:
- EP-A- 1 504 724
- US-B1- 6 272 371
- US-B1- 6 626 832

## Description

### BACKGROUND

### 1. Technical Field

The present disclosure relates generally to an ultrasonic dissection and coagulation system for surgical use. More specifically, the present disclosure relates to an ultrasonic instrument including a detection circuit for detecting deflection of an ultrasonic probe.

### 2. Background of Related Art

Ultrasonic instruments for surgical use and the benefits associated therewith are well known. For example, the use of an ultrasonic generator in conjunction with a surgical scalpel facilitates faster and easier cutting of organic tissue and accelerates coagulation. Improved cutting results from increased body tissue to scalpel contact caused by the high frequency of vibration of the scalpel blade with respect to body tissue. Improved coagulation results from heat generated by contact between the scalpel blade and the body tissue as the scalpel blade is vibrated at a high frequency.

Conventional ultrasonic instruments include a variety of probes (e.g., cutting blades, shears, hook, ball, etc.) adapted for specific medical procedures. The ultrasonic probe is disposed at a distal end, the end furthest away from the surgeon, of the ultrasonic instrument. These ultrasonic instruments are primarily used in medical procedures involving endoscopic procedures, in which the surgeon has limited visualization of the position of the probe relative to surrounding tissue. As a result there is a risk that the probe will come in contact with thick tissue or other obstructions which will overstress the probe and may break the probe off of the ultrasonic instrument Such stress does not only damage expensive medical equipment but can also cause extraneous debris (e.g., broken off tip of the probe) to contaminate the surgical site. Such an instrument is disclosed in EP 1 504 724 A1.

Therefore there is a need for an ultrasonic apparatus which alerts the surgeon to overstresses exerted on the probe to prevent damage thereto.

### SUMMARY

The present disclosure provides for an ultrasonic instrument having an ultrasonic probe and a deflection detection circuit. The deflection circuit includes a secondary power source which supplies electrical current to the ultrasonic probe, a tube, and to a visual and/or audio alarm which notifies the surgeon when the ultrasonic probe is overstressed. This occurs when the probe comes into contact with the tube positioned to gauge overstress in the probe thereby closing the detection circuit.

According to an embodiment of the present disclosure, an ultrasonic surgical instrument is provided. The instrument includes an ultrasonic probe configured to conduct electricity. The ultrasonic probe is positioned a predetermined distance from one or more tubes. The ultrasonic probe is operatively connected to an ultrasonic generator for vibration. The instrument also includes a deflection detection circuit having a secondary power source and an indicator, the power source is configured to supply electrical current to the tube, the probe, and the indicator, wherein the circuit is configured to close in response to the probe contacting the tube when the probe is deflected toward the tube thereby activating the alarm.

According to another aspect of the present disclosure an ultrasonic surgical instrument is disclosed. The instrument includes an ultrasonic probe which is positioned a predetermined distance from at least one tube. The ultrasonic probe is adapted to be operatively connected to a transducer for vibration. The instrument also includes a deflection detection circuit which includes a secondary power source, a magnetic proximity sensor and an indicator. The power source is configured to supply electrical current to the magnetic proximity sensor indicator, wherein the magnetic proximity sensor is adapted to sense deflection of the ultrasonic probe and to activate the indicator in response thereto.

According to a further aspect of the present disclosure, an ultrasonic surgical instrument is disclosed. The instrument includes an ultrasonic probe configured to conduct electricity extending from an elongated vibration coupler. The ultrasonic probe is positioned a predetermined distance from at least one tube. The vibration coupler is adapted to be operatively connected to a transducer for vibration. The instrument also includes a deflection detection circuit which includes a secondary power source, an impedance sensor and an indicator. The power source is configured to supply electrical current to the ultrasonic probe and the indicator, wherein the impedance sensor is adapted to sense deviation in impedance of the ultrasonic probe from a predetermined threshold and to activate the indicator in response thereto.

### BRIEF DESCRIPTION OF THE DRAWINGS

The above and other aspects, features, and advantages of the= present disclosure will become more apparent in light of the following detailed description when taken in conjunction with the accompanying drawings in which:
Fig. 1 is a perspective view of the ultrasonic dissection and coagulation system with the ultrasonic instrument inserted partially through a cannula;
Fig. 2 is a perspective view with parts separated of the clamp of the ultrasonic instrument of Fig. 1;
Fig. 3 is a perspective view with parts separated of the elongated body portion of the ultrasonic instrument of Fig. 1; and
Fig. 4 is a perspective view with parts separated of the ultrasonic instrument of Fig. 1;
Fig. 5 is a perspective view with parts separated of the rotation assembly of the ultrasonic instrument of Fig. 1;
Fig. 6 is a cross-sectional schematic view of the ultrasonic instrument of Fig. 1 illustrating one embodiment of the present disclosure;
Fig. 7 is a cross-sectional schematic view of the ultrasonic instrument of Fig. 1 illustrating another embodiment of the present disclosure;
Fig. 8 is a cross-sectional schematic view of the ultrasonic instrument of Fig. 1 illustrating another embodiment of the present disclosure; and
Fig. 9 is a schematic view of the ultrasonic instrument of Fig. 1 illustrating another embodiment of the present disclosure.

### DETAILED DESCRIPTION

Preferred embodiments of the present disclosure will be described herein below with reference to the accompanying drawings. In the following description, well-known functions or constructions are not described in detail to avoid obscuring the present disclosure in unnecessary detail. As used herein, the term "distal" refers to that portion which is further from the user while the term "proximal" refers to that portion which is closer to the user or surgeon.

The present disclosure provides for an ultrasonic instrument having a deflection detection circuit which activates an alert, which may be tactile, audible and/or visual, when an ultrasonic probe is overstressed, such as when the probe is being used outside its normal operational range or a predetermined moment is exerted thereon. When stress is exerted, the probe comes in contact with a tube or other adjacent structure (e.g., a tubular body, a contact, etc.). An electrical current supplied by a secondary power source is passed through the probe and the outer tube. Consequently the probe acts as a switch and closes the detection circuit and activates the alert.

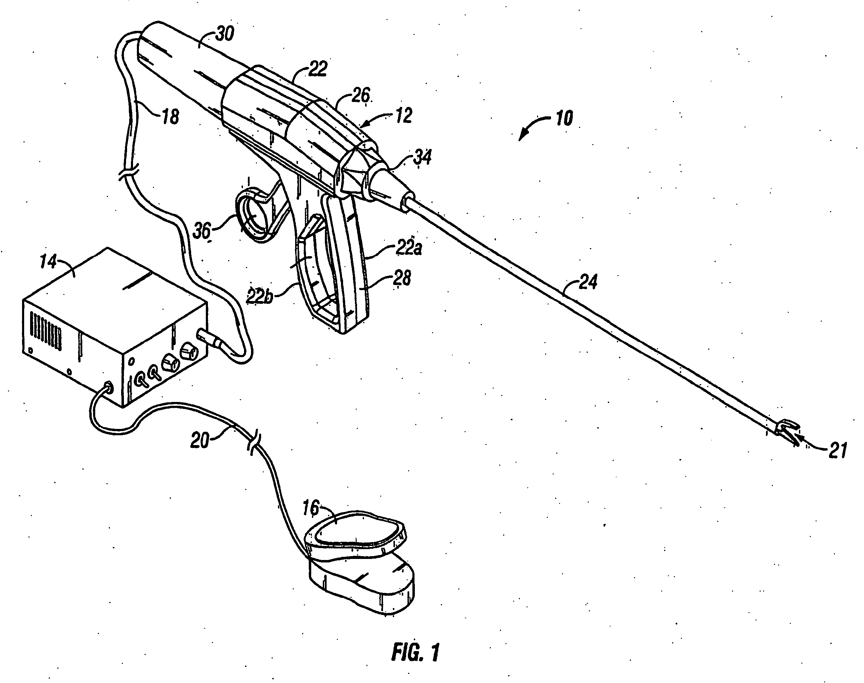

Fig. 1 illustrates the ultrasonic dissection and coagulation system shown generally as 10. The dissection and coagulation system 10 includes an ultrasonic instrument 12, a generator module 14, and a remote actuator 16. Generator module 14 is operatively connected to ultrasonic instrument 12 by an electrically conductive cable 18 and functions to control the power and frequency of current supplied to ultrasonic instrument 12. Any suitable controller capable of delivering power to ultrasonic instrument 12 can be used. Remote actuator 16, e.g., pedal actuator, is operatively connected to generator module 14 by electrically conductive cable 20 and can be actuated to initiate the supply of power to ultrasonic instrument 12 via generator module 14 to effect vibratory motion of ultrasonic instrument 12 to cut and coagulate tissue.

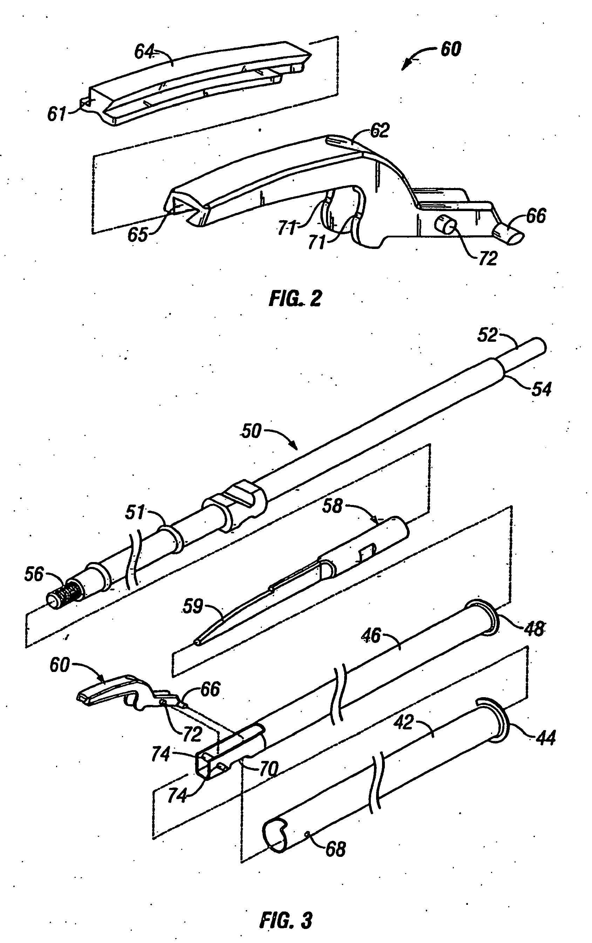

The ultrasonic instrument 12 includes housing 22 and elongated body portion 24 extending distally therefrom. Housing 22 is preferably formed from molded housing half-sections 22a and 22b and includes a barrel portion 26 having a longitudinal axis aligned with the longitudinal axis of body portion 24 and a stationary handle portion 28 extending obliquely from barrel portion 26. Ultrasonic transducer 30 is supported within and extends from the proximal end of housing 22 and is connected to generator module 14 via cable 18. The transducer 30 can be a separate component or incorporated into the ultrasonic instrument 12. The generator module 14 supplies electrical energy having ultrasonic frequency to the transducer 30 to cause oscillation thereof The transducer 30, which may be one of a variety of electromechanical types, e.g., electrodynamic, piezoelectric, magnetostrictive, is connected to a an ultrasonic probe 21 (Fig. 3) to cause oscillation thereof.

The ultrasonic probe 21 extends through the elongated body portion 24. Movable handle 36 and stationary handle portion 28 may include openings 38 and 40, respectively, to facilitate gripping and actuation of ultrasonic instrument 12. Elongated body portion 24 is supported within rotatable knob 34 and may be selectively rotated by rotating knob 34 with respect to housing 22 to change the orientation of the distal end of ultrasonic instrument 12.

Those skilled in the art will understand that the ultrasonic probe 21 is an illustrative embodiment of an ultrasonic probe and that other types and/or forms of ultrasonic implements are envisioned; such as a blade, a hook, or a ball, and/or an aspirator assembly. An example of an ultrasonic aspirator instrument is shown and described in commonly-owned U.S. Patent No. 4,922,902 entitled "METHOD FOR REMOVING CELLULAR MATERIAL WITH ENDOSCOPIC ASPIRATOR".

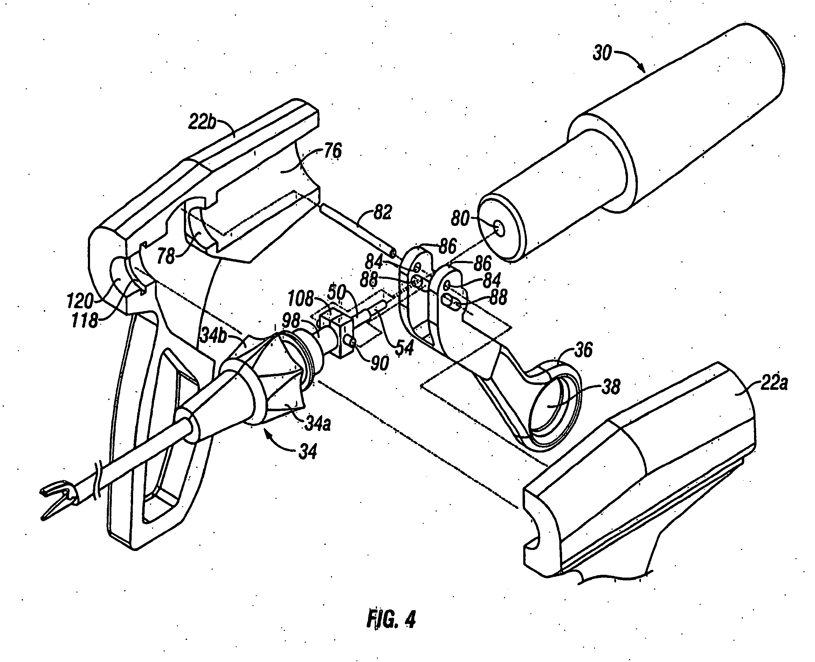

Figs. 2 and 3 illustrate elongated body portion 24 with parts separated. Elongated body portion 24 includes an outer tube 42 which is preferably cylindrical and has a proximally located annular flange 44 dimensioned to engage rotatable knob 34 (Fig. 1) as described below. An elongated actuator tube 46, which is also preferably cylindrical, is configured to be slidably received within outer tube 42 and includes a proximally located annular flange 48 dimensioned to engage coupling member 98 (Fig. 4) which is supported within housing 22 (Fig. 1) and will be described in detail below. Ultrasonic probe 21 includes an elongated coupler 50 which is dimensioned to extend through elongated actuator tube 46 and a cutting jaw 58. A proximal end 52 of the elongated coupler 50 has a reduced diameter portion 54 configured to engage the transducer 30 (Fig. 4) and a distal end 56 adapted to be operatively connected to the cutting jaw 58. In other embodiments, the ultrasonic probe 21 is formed in a single, rather than multiple parts. A plurality of silicon rings 51 can be molded or otherwise attached to the nodal points along ultrasonic probe 21 to seal between ultrasonic probe 21 and actuator tube 46. Preferably, cutting jaw 58 includes an internal proximal threaded bore (not shown) which is dimensioned to receive threaded distal end 56 of ultrasonic probe 21. Alternately, cutting jaw 58 can be formed integrally with elongated coupler 50, cutting jaw 58 may include a threaded proximal end configured to be received within a threaded bore formed in elongated coupler 50, or other attachment devices can be used.

A clamp 60 having a clamp body 62 and a tissue contact member 64 secured to clamp body 62 is operatively connected to the distal end of outer tube 42 and actuator tube 46. Clamp body 62 includes a pair of tissue engaging stops 71 at the proximal end of the exposed blade surface 59. Tissue contact member 64 is preferably composed of Teflon and is preferably fastened to clamp body 62 by a tongue and groove fastening assembly (reference numerals 61 and 65, respectively), although other fastening assemblies are also envisioned. Tissue contact member 64 functions to isolate clamp 60, which is preferably metallic, from jaw 58, which is also preferably metallic, to prevent metal to metal contact.

Tissue contact member 64 also functions to grip tissue to prevent movement of the tissue with vibrating cutting jaw 58. Alternately, at least one row of teeth may be positioned on clamp 60 to grip tissue. Pivot members, here shown as pins 66, located at the proximal end of clamp body 62, are configured to be received within openings 68 formed in the distal end of outer tube 42. A guide slot 70 formed in the distal end of the actuator tube 46 permits relative movement between actuator tube 46 and clamp body 62 by allowing the actuator tube 46 to move in relation to pins 66. A pair of camming members, here shown as protrusions 72, are also formed on clamp body 62 and are positioned to be received within cam slots 74 formed in the distal end of actuator tube 46. Movement of actuator tube 46 and clamp 60 will be described in detail below.

Cutting jaw 58 includes a curved blade surface 59 that slopes downwardly and outwardly in the distal direction and may include a cutting edge. Preferably, the entire blade surface 59 exposed to tissue, i.e., the portion of blade surface 59 between tissue engaging stops 71 and the distal end of blade surface 59, has a tangent which defines an angle with respect to the longitudinal axis of elongated body portion 24 that varies along the length of blade surface 59 from about 5 degrees to about 75 degrees. Ideally, the angle defined by a line tangent to the blade surface and the longitudinal axis of elongated body portion 24 varies from about 5 degrees to about 45 degrees along the length of the blade surface. The curved blade surface provides better visibility at the surgical site. Clamp 60 is movable from an open position in which tissue contact member 64 is spaced from blade surface 59 to a clamped position in which tissue contact member is juxtaposed with and in close alignment with blade surface 59 to clamp tissue therebetween. The interior surface of tissue contact member 64 is curved to correspond to blade surface 59. Actuation of clamp 60 from the open position to the clamped position will be described in detail below.

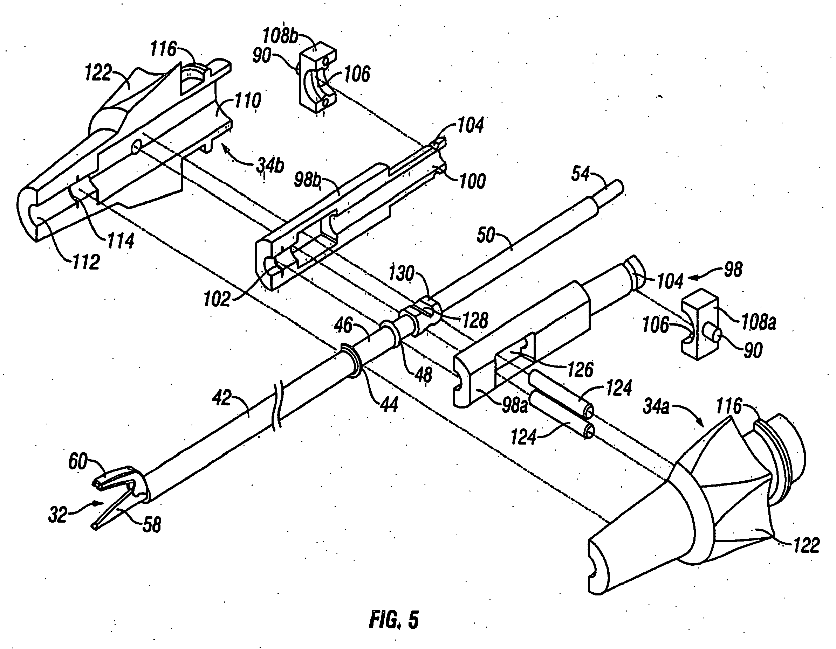

Referring now to Figs. 4 and 5, the handle assembly and the rotation assembly will now be discussed. Housing half-sections 22a and 22b define a chamber 76 configured to receive a portion of ultrasonic transducer 30. Chamber 76 has an opening 78 communicating with the interior of housing 22. Ultrasonic transducer 30 includes a bore 80 configured to receive proximal end 52 of ultrasonic probe 21. In the assembled condition, proximal end 52 extends through opening 78 into bore 80. Ultrasonic transducer 30 may be secured to vibration coupler 50 using any known attachment apparatus, such as a torque wrench. As disclosed therein, the proximal end of transducer 30 may be configured to engage the torque wrench. Movable handle 36 is pivotally connected between housing half sections 22a and 22b about pivot pin 82 which extends through holes 84 formed in legs 86 of movable handle 36. A cam slot 88 formed in each leg 86 is configured to receive a protrusion 90 projecting outwardly from coupling member 98 (Fig. 5).

As illustrated in Fig. 5, coupling member 98 operatively connects movable handle 36 to actuator tube 46 and is preferably formed from molded half sections 98a and 98b to define a throughbore 100 dimensioned to slidably receive the proximal end of ultrasonic probe 21. Coupling member 98 has an inner distally located annular groove 102 dimensioned to receive annular flange 48 of actuator tube 46 and an outer proximally located annular groove 104. Groove 104 is positioned to receive an annular rib 106 formed on the internal wall of a swivel member 108 (Fig. 4). Swivel member 108 is preferably formed from molded half-sections 108a and 108b and permits rotation of coupling member 98 relative to movable handle 36. Protrusions 91 project outwardly from sidewalls of swivel member 108 and extend through cam slots 88 of movable handle 36 (Fig. 4).

Referring to Figs. 4 and 5, rotation knob 34 is preferably formed from molded half-sections 34a and 34b and includes a proximal cavity 110 for slidably supporting coupling member 98 and a distal bore 112 dimensioned to receive outer tube 42. An annular groove 114 formed in bore 112 is positioned to receive annular flange 44 of outer tube 42. The outer wall of knob 34 has a proximally located annular ring 116 dimensioned to be rotatably received within annular slot 118 formed in opening 120 of housing 22. The outer wall of knob 34 also includes scalloped surface 122 to facilitate gripping of rotatable knob 34. Annular ring 116 permits rotation of knob 34 with respect to housing 22 while preventing axial movement with respect thereto. A pair of cylindrical rods 124 extend between half-sections 34a and 34b through a rectangular opening 126 formed in coupling member 98. Rods 124 engage a pair of concave recesses 128 formed in fitting 130 of ultrasonic probe 21, such that rotation of knob 34 causes rotation of ultrasonic probe 21 and thus rotation of jaw 58 and clamp 60. Alternately, recesses 128 can be monolithically formed with ultrasonic probe 21.

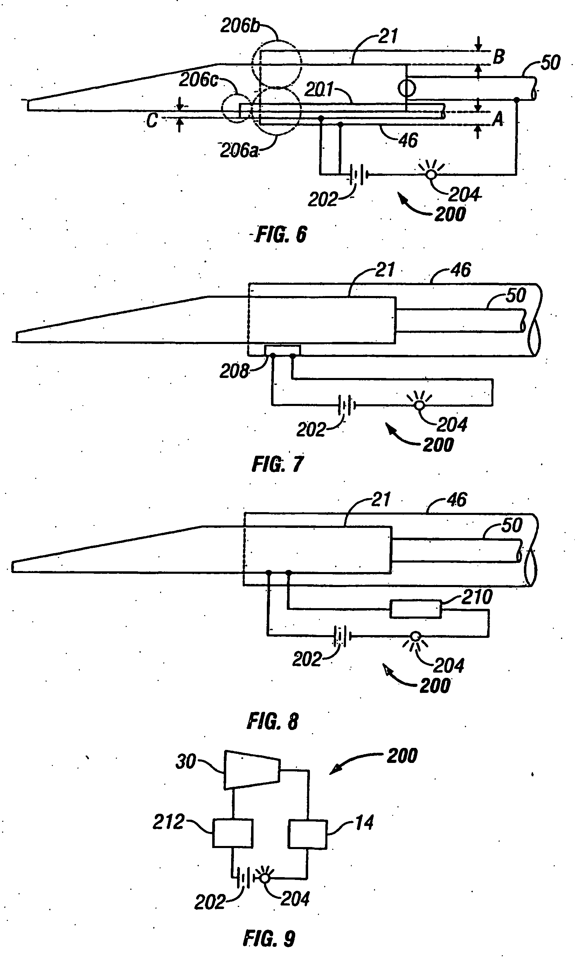

With reference to Fig. 6, disposed a predetermined distance away from the ultrasonic probe 21 is a contact structure 201 which is configured to conduct electricity and is preferably formed from a medical grade conductive material such as stainless steel, titanium, etc. The contact structure 201 has a shape of a contact strip and is positioned a predetermined distance from about 1 mm to about 4 mm from the ultrasonic probe 21 and can be positioned on any side thereof. It is also envisioned that more than one contact structure 201 may be positioned around the ultrasonic probe 21. It is further envisioned that the contact structure 201 can have a plurality of shapes and forms (e.g., curved strip, a wire; etc.). It is further envisioned that the actuator tube 46 may be used in place of the contact strip 201 and perform the functionality thereof.

Fig. 6 shows a deflection detection circuit 200 which includes a secondary power source 202 and a visual alarm indicator 204. It is envisioned that in one embodiment the detection circuit 200 is an electrical circuit between the actuator tube 46 as well as the ultrasonic probe 21 all of which are connected to the power source 202. When the ultrasonic probe 21 is overstressed it would come in contact with the contact structure 201 and/or the actuator tube 46 and thereby closing the circuit and tripping off the indicator 204.

In another embodiment, the detection circuit 200 includes the power source 202 which supplies electrical energy to the contact structure 201 and at least the ultrasonic probe 21. In this embodiment, the overstressed probe 21 comes in contact with the contact structure 201 and not actuator tube 46.

The power source 202 may be DC power supply electrically connected to the generator module 14 or a stand-alone battery. In addition, the power source 202 is configured to supply a low voltage current which is sufficient to power the alarm indicator 204 but not large enough to interfere with the primary power supplied to the ultrasonic probe 21 by the generator 25 (e.g., electrocute the patient). Those skilled in the art will readily appreciate the voltage range suitable for this purpose. The power source 202 may be stand alone or be included within the generator module 14.

The alarm indicator 204 may be a light emitting device, such as a light emitting diode or a light bulb embedded in the housing portion 18. The alarm indicator 204 is activated when the detection circuit 200 is closed, which occurs when the probe 21 comes in contact with the tubular body. Those skilled in the art will appreciate that the visual alarm indicator 204 may be substituted by an audio alarm (e.g., a speaker) or another alarm device, such as a tactile alarm device (e.g., a vibrating mechanism [not explicitly shown] disposed within the housing portion 18).

With reference to the first embodiment, the actuator tube 46 as well as the ultrasonic probe 21 are not in physical contact during normal operation of the instrument 10 (e.g., when the ultrasonic probe 21 is not overstressed). In addition, the actuator tube 46 and the ultrasonic probe 21 are electrically isolated because they are kept separate by silicon rings 51. As a result, the detection circuit 200 is open and the alarm indicator 204 is not active during normal operation of the instrument 10.

With reference to Fig. 6, when the ultrasonic probe 21 is overstressed it comes in contact with the inner surface of the actuator tube 46. During normal operation, the ultrasonic probe 21 is separated from the actuator tube 46 by a gap distance A and gap distance B on the bottom and top portions of the actuator tube 46, respectively. The gap distances A, B can be from about 1 mm to about 4 mm. Once the ultrasonic probe 21 is overstressed the ultrasonic probe 21 contacts the inner surface of the outer tube 42. For instance, if downward pressure is applied, the ultrasonic probe 21 will contact the actuator tube 46 at a point 206a. Similarly, when upward pressure is applied, the ultrasonic probe 21 will make contact at a point 206b. Those skilled in the art will appreciate that the ultrasonic probe 21 may be tilted in any direction depending on the pressure exerted thereon and that points 206a, 206b are illustrative.

The ultrasonic probe 21 contacts the inner surface of the actuator tube 46 when sufficient pressure is exerted on the ultrasonic probe 21 thereby closing the detection circuit 200, which activates the alarm indicator 204. This alerts the surgeon that the ultrasonic probe 21 is overstressed and that the present usage of the instrument 10 must seize to avoid damaging and/or breaking off the ultrasonic probe 21.

With reference to the second embodiment, the contact structure 201 and the ultrasonic probe 21 are not in physical contact during normal operation of the ultrasonic probe 21 and are, thus, electrically isolated from one another. When the ultrasonic probe 21 is overstressed (e.g., the probe 21 is operating outside the normal parameters) this may result in oscillation movements which are outside the normal range. Overstress may be also the result of a large moment exerted on the probe. During the normal operation, the maximum range to which the ultrasonic probe 21 may tilt is expressed by the gap distance C, the distance between the ultrasonic probe 21 and the contact structure 201, which is from about 1 millimeter to about 4 millimeters. The normal operational parameters are surpassed when the ultrasonic probe 21 is overstressed, thus, the ultrasonic probe 21 tilts toward the contact structure 201, closing the gap distance C at a point 206c. The detection circuit 200 also closes and supplies power to the alarm indicator 204.

It is further envisioned that there may be more than one contact structure 201 positioned around the ultrasonic probe 21 to facilitate in deflection detection.

Fig. 7 shows another embodiment of the detection circuit 200 which includes a magnetic proximity sensor 208 connected to the power source 202 and the alarm indicator 204. The magnetic proximity sensor 208 is disposed near the ultrasonic probe 21. The magnetic proximity sensor 208 is calibrated to detect when the ultrasonic probe 21 vibrate outside their prescribed movement ranges, which results in the ultrasonic probe 21 approaching the magnetic proximity sensor 208. In response thereto, the magnetic proximity sensor 208 triggers the alarm indicator 204.

Fig. 8 shows another embodiment of the detection circuit 200 which includes an impedance sensor 210 connected to the ultrasonic probe 21. The impedance sensor 210 measures impedance within the ultrasonic probe 21. This may be accomplished by allowing a low voltage current to flow through the ultrasonic probe 21 (e.g., via the power source 202). The impedance sensor 210 measures the impedance based on the voltage and current signals being passed through the ultrasonic probe 21. During normal operation, the impedance of the ultrasonic probe 21 remains within a predetermined range. If the ultrasonic probe 21 is operating outside normal parameters, such as the ultrasonic probe 21 is overheating (e.g., due to stress), the impedance thereof changes as well since impedance varies with temperature. The impedance sensor 210 is calibrated to sense such changes in impedance and once detected, the impedance sensor 210 signals the alarm indicator 204.

Fig. 9 shows one other embodiment of the detection circuit 200 which includes a sensor circuit 212 connected to the ultrasonic transducer 30 and the generator module 14. The sensor circuit 212 is adapted to measure a variety of electrical parameters within the ultrasonic transducer 30 and the generator module 14. The sensor circuit 212 is configured to measure internal voltage, current, power, and frequency of the generator module 14. During abnormal operation of the ultrasonic probe 21 the voltage drops across the piezoelectric stack of the ultrasonic transducer 30. Further, the frequency, power, voltage and current of the generator module 14 also fluctuate when the ultrasonic probe 21 is operating outside normal parameters. The sensor circuit 212 detects deviations in voltage, frequency and power in the ultrasonic transducer 30 and the generator module 14 and activates the alarm indicator 204.

## Claims

1. An ultrasonic surgical instrument (12) comprising:
an ultrasonic probe (21) configured to conduct electricity and at least one tube (46), the ultrasonic probe being positioned a predetermined distance from the at least one tube and adapted to be operatively connected to an ultrasonic generator (14) for vibration; and
a deflection detection circuit (200) including a secondary power source (202) and an indicators (204), the secondary power source being configured to supply electrical current to the at least one tube, the ultrasonic probe, and the indicator, wherein the circuit is configured to close in response to the ultrasonic probe contacting the at least one tube when the ultrasonic probe is deflected toward the at least one tube thereby activating the indicator.

2. An ultrasonic surgical instrumentas-in claim 1, wherein the at least one tube is formed from a medical grade electrically conductive material selected from the group consisting of stainless steel-and titanium.

3. An ultrasonic surgical instrument as in claim 1, wherein the ultrasonic probe is formed from a medical grade electrically conductive Material selected from the group consisting of stainless steel and titanium.

4. An ultrasonic surgical instrument as in claim 1, wherein the at least one tube is an elongated tube configured to conduct electricity extending from the housing defining a longitudinal axis and having proximal and distal portions and a lumen extending Wherethrough; wherein the ultrasonic probe is disposed within the lumen.

5. An ultrasonic surgical instrument as in claim 4, wherein the instrument further comprises:
a housing (22); and
a rotation knob (34) positioned adjacent to the housing, the ultrasonic probe and the at least one tube being operatively connected to the rotation knob, the rotation knob rotatable to rotate the at least one tube and the ultrasonic probe about the longitudinal axis to change their orientation with respect to tissue.

6. An ultrasonic surgical instrument as in claim 4, wherein the instrument further comprises a transducer (30) removably connected to the Sousing.

7. An ultrasonic-surgical instrument as in claim 6, wherein the transducer is formed from a material selected from the group consisting of electrodynamic, piezoelectric, and magnetostrictive materials.

8. An ultrasonic surgical instrument as in claim 1, wherein the indicator is a visual alarm.

9. An ultrasonic surgical instrument-as-in claim-8, wherein the visual alarm is selected from the group consisting of a light emitting diode and a light bulb.

10. An ultrasonic surgical instrument as in claim 1, wherein the indicator is an audio alarm

11. An ultrasonic surgical instrument as in claim 10, wherein the audio alarm is a speaker.

12. An-ultrasonic surgical instrument as in claim 1, wherein the indicator is a tactile alarm.

13. An ultrasonic surgical instrument as in claim 1, wherein the secondary power source is a DC power source.

14. An ultrasonic surgical instrument as in claim 13, wherein the DC powersource is a battery.

15. An ultrasonic surgical instrument as in claim 13, wherein the DC power source is electrically connected to the ultrasonic generator.

16. An ultrasonic surgical instrument as in claim 1, wherein the probe is selected from the group consisting one of a blade, a shears, a hook, a ball, and an aspirator.

17. An ultrasonic instrument as in claim 1, wherein the predetermined distance is from about 1 millimeter to about 4 millimeters.

18. An ultrasonic instrument as in claim 1, wherein the tube comprises a contact structure in the form of one of a strip and a wire.

19. An ultrasonic surgical instrument comprising:
an ultrasonic probe and an outer tube, the ultrasonic probe being positioned a predetermined distance from the at least one outer tube and adapted to be operatively connected to a transducer for vibration; and
a deflection detection circuit including a secondary power source, a magnetic proximity sensor (208) and an indicator, the secondary power source being configured to supply electrical current to the magnetic proximity sensor and the indicator, wherein the magnetic proximity sensor is adapted to sense deflection of the ultrasonic probe and to activate the indicator in response thereto.

20. An ultrasonic surgical instrument comprising:
an ultrasonic probe configured to conduct electricity and at least one tube, the ultrasonic probe being positioned a predetermined distance from the at least one tube and adapted to be operatively connected to a transducer for vibration; and
a deflection detection circuit including a secondary power source, an impedance sensor (210) and an indicator, the secondary power source being configured to supply electrical current to the ultrasonic probe and the indicator, wherein the impedance sensor is adapted to sense deviation in impedance of-the ultrasonic probe from a predetermined threshold and to activate the indicator in response thereto.

## Patentansprüche

1. Chirurgisches Ultraschallinstrument (12) mit:
einer Ultraschallsonde (21) mit einem Aufbau zum Leiten von Elektrizität und mindestens einem Rohr (46), wobei die Ultraschallsonde in einem bestimmten Abstand von dem mindestens einen Rohr angeordnet und geeignet ausgestaltet ist, um betrieblich mit einem Ultraschallgenerator (14) für eine Vibration verbunden zu sein; und
einer Ablenkungserfassungsschaltung (200), welche eine sekundäre Stromquelle (202) und eine Anzeigeeinrichtung (204) einschließt, wobei die zweite Stromquelle ausgestaltet ist, um elektrischen Strom zu dem mindestens einen Rohr, der Ultraschallsonde und der Anzeigeeinrichtung zu liefern, und die Schaltung konfiguriert ist, um in Abhängigkeit von der Ultraschallsonde unter Berühren des mindestens einen Rohres zu blockieren, wenn die Ultraschallsonde zu dem mindestens einen Rohr hin abgelenkt wird, wodurch die Anzeigeeinrichtung angesteuert wird.

2. Chirurgisches Ultraschallinstrument nach Anspruch 1, wobei das mindestens eine Rohr aus einem medizinisch elektrisch leitenden Material geformt ist, welches aus der Gruppe ausgewählt ist, die aus Edelstahl und Titan besteht.

3. Chirurgisches Ultraschallinstrument nach Anspruch 1, wobei die Ultraschallsonde aus einem medizinisch elektrisch leitenden Material gebildet ist, welches aus der Gruppe ausgewählt ist, die aus Edelstahl und Titan besteht.

4. Chirurgisches Ultraschallinstrument nach Anspruch 1, wobei das mindestens eine Rohr ein längliches Rohr mit einer Ausgestaltung ist, um Elektrizität zu leiten, die sich von dem Gehäuse ausdehnt, das unter Bildung einer Längsachse und mit proximalen und distalen Abschnitten und einem sich durch diese erstreckenden Lumen gebildet ist, wobei die Ultraschallsonde in dem Lumen angeordnet ist.

5. Chirurgisches Ultraschallinstrument nach Anspruch 4, wobei das Instrument ferner aufweist:
ein Gehäuse (22) und
einen Drehknopf (34) mit einer Anordnung neben dem Gehäuse, wobei die Ultraschallsonde und das mindestens eine Rohr betrieblich mit dem Drehknopf verbunden sind, der drehbar ist, um das mindestens eine Rohr und die Ultraschallsonde um die Längsachse zu drehen und ihre Ausrichtung bezüglich des Gewebes zu ändern.

6. Chirurgisches Ultraschallinstrument nach Anspruch 4, wobei das Instrument ferner einen lösbar mit dem Gehäuse verbundenen Wandler (30) aufweist.

7. Chirurgisches Ultraschallinstrument nach Anspruch 6, wobei der Wandler aus einem Material gebildet ist, welches aus der Gruppe ausgewählt ist, die aus elektrodynamischen, piezoelektrischen und magnetostriktiven Materialien besteht.

8. Chirurgisches Ultraschallinstrument nach Anspruch 1, wobei die Anzeigeeinrichtung eine visuelle Alarmvorrichtung ist.

9. Chirurgisches Ultraschallinstrument nach Anspruch 8, wobei die visuelle Alarmvorrichtung aus der Gruppe ausgewählt ist, die aus einer lichtemittierenden Diode und einer Glühlampe besteht.

10. Chirurgisches Ultraschallinstrument nach Anspruch 1, wobei die Anzeigeeinrichtung eine akustische Alarmvorrichtung ist.

11. Chirurgisches Ultraschallinstrument nach Anspruch 10, wobei die akustische Alarmvorrichtung ein Lautsprecher ist.

12. Chirurgisches Ultraschallinstrument nach Anspruch 1, wobei die Anzeigeeinrichtung eine Alarmvorrichtung mit taktiler Wahrnehmung ist.

13. Chirurgisches Ultraschallinstrument nach Anspruch 1, wobei die zweite Stromquelle eine Gleichstromquelle ist.

14. Chirurgisches Ultraschallinstrument nach Anspruch 13, wobei die Gleichstromquelle eine Batterie ist.

15. Chirurgisches Ultraschallinstrument nach Anspruch 13, wobei die Gleichstromquelle mit dem Ultraschallgenerator elektrisch verbunden ist.

16. Chirurgisches Ultraschallinstrument nach Anspruch 1, wobei die Sonde aus der Gruppe ausgewählt ist, die aus einer Klinge, einer Schere, einem Haken, einer Kugel und einem Saugapparat besteht.

17. Ultraschallinstrument nach Anspruch 1, wobei der bestimmte Abstand von etwa 1 mm bis etwa 4 mm beträgt.

18. Ultraschallinstrument nach Anspruch 1, wobei das Rohr eine Kontaktstruktur in der Form eines Bandes und eines Drahtes aufweist.

19. Chirurgisches Ultraschallinstrument mit:
einer Ultraschallsonde und einem äußeren Rohr, wobei die Ultraschallsonde in einem vorbestimmten Abstand von dem mindestens einen äußeren Rohr angeordnet und geeignet ausgestaltet ist, und im Betrieb mit einem Wandler für die Vibration verbunden ist, und
einer Ablenkungserfassungsschaltung, die eine zweite Stromquelle, einen magnetischen Annäherungsschalter (208) und eine Anzeigeeinrichtung einschließt, wobei die zweite Stromquelle ausgestaltet ist, um elektrischen Strom zu dem magnetischen Annäherungsschalter und der Anzeigeeinrichtung zu liefern, wobei der magnetische Annäherungsschalter geeignet ausgestaltet ist, um die Ablenkung der Ultraschallsonde abzufühlen und die Anzeigeeinrichtung in Abhängigkeit davon anzusteuern.

20. Chirurgisches Ultraschallinstrument mit:
einer Ultraschallsonde mit einer Ausgestaltung, um Elektrizität und mindestens ein Rohr zu führen, wobei die Ultraschallsonde in einem vorbestimmten Abstand von dem mindestens einen Rohr angeordnet und geeignet ausgestaltet ist, um im Betrieb mit einem Wandler für die Vibration verbunden zu sein, und
einer Ablenkungserfassungsschaltung mit einer zweiten Stromquelle, einem Impedanzsensor (210) und einer Anzeigeeinrichtung, wobei die zweite Stromquelle ausgestaltet ist, um elektrischen Strom zu der Ultraschallsonde und der Anzeigeeinrichtung zu liefern, und der Impedanzsensor geeignet ausgestaltet ist, um die Abweichung der Impedanz der Ultraschallsonde von einem vorbestimmten Schwellwert abzufühlen und die Anzeigeeinrichtung in Abhängigkeit davon anzusteuern.

## Revendications

1. Instrument chirurgical (12) à ultrasons comportant :
une sonde à ultrasons (21) configurée pour conduire l'électricité, et au moins un tube (46), la sonde à ultrasons étant positionnée à une distance prédéterminée du, au moins un, tube et étant conçue pour être connectée fonctionnellement à un générateur (14) d'ultrasons à des fins de vibration ; et
un circuit (200) de détection de déviation comprenant une source d'énergie secondaire (202) et un indicateur (204), la source d'énergie secondaire étant configurée pour fournir du courant électrique au, au moins un, tube, à la sonde à ultrasons et à l'indicateur, dans lequel le circuit est configuré pour se fermer en réponse à l'entrée en contact de la sonde à ultrasons avec le, au moins un, tube lorsque la sonde à ultrasons est déviée vers le, au moins un, tube, activant ainsi l'indicateur.

2. Instrument chirurgical à ultrasons selon la revendication 1, dans lequel le, au moins un, tube est formé d'une matière électriquement conductrice de qualité médicale choisie dans le groupe constitué de l'acier inoxydable et du titane.

3. Instrument chirurgical à ultrasons selon la revendication 1, dans lequel la sonde à ultrasons est formée d'une matière électriquement conductrice de qualité médicale choisie dans le groupe constitué de l'acier inoxydable et du titane.

4. Instrument chirurgical à ultrasons selon la revendication 1, dans lequel le, au moins un, tube est un tube allongé configuré pour conduire l'électricité, s'étendant depuis le boîtier définissant un axe longitudinal et ayant des parties proximale et distale et une lumière s'étendant entre elles, dans lequel la sonde à ultrasons est disposée à l'intérieur de la lumière.

5. Instrument chirurgical à ultrasons selon la revendication 4, lequel instrument comporte en outre :
un boîtier (22) ; et
un bouton tournant (34) positionné de façon à être adjacent au boîtier, la sonde à ultrasons et le, au moins un, tube étant reliés fonctionnellement au bouton tournant, le bouton tournant pouvant être tourné pour faire tourner le, au moins un, tube et la sonde à ultrasons autour de l'axe longitudinal afin de modifier leur orientation par rapport à des tissus.

6. Instrument chirurgical à ultrasons selon la revendication 4, lequel instrument comporte en outre un transducteur (30) relié de façon amovible au boîtier.

7. Instrument chirurgical à ultrasons selon la revendication 6, dans lequel le transducteur est formé d'une matière choisie dans le groupe constitué de matières électrodynamiques, piézoélectriques et magnétostrictives.

8. Instrument chirurgical à ultrasons selon la revendication 1, dans lequel l'indicateur est une alarme visuelle.

9. Instrument chirurgical à ultrasons selon la revendication 8, dans lequel l'alarme visuelle est choisie dans le groupe constitué d'une diode émettrice de lumière et d'une ampoule lumineuse.

10. Instrument chirurgical à ultrasons selon la revendication 1, dans lequel l'indicateur est une alarme sonore.

11. Instrument chirurgical à ultrasons selon la revendication 10, dans lequel l'alarme sonore est un haut-parleur.

12. Instrument chirurgical à ultrasons selon la revendication 1, dans lequel l'indicateur est une alarme tactile.

13. Instrument chirurgical à ultrasons selon la revendication 1, dans lequel la source d'énergie secondaire est une source d'énergie à courant continu.

14. Instrument chirurgical à ultrasons selon la revendication 13, dans lequel la source d'énergie à courant continu est une batterie.

15. Instrument chirurgical à ultrasons selon la revendication 13, dans lequel la source d'énergie à courant continu est connectée électriquement au générateur d'ultrasons.

16. Instrument chirurgical à ultrasons selon la revendication 1, dans lequel la sonde est choisie dans le groupe consistant en l'un d'une lame, de cisailles, d'un crochet, d'une bille et d'un aspirateur.

17. Instrument à ultrasons selon la revendication 1, dans lequel la distance prédéterminée va d'environ 1 mm à environ 4 millimètres.

18. Instrument à ultrasons selon la revendication 1, dans lequel le tube comporte une structure de contact sous la forme d'une bande et d'un fil.

19. Instrument chirurgical à ultrasons comportant :
une sonde à ultrasons et un tube extérieur, la sonde à ultrasons étant positionnée à une distance prédéterminée du, au moins un, tube extérieur et étant conçue pour être reliée fonctionnellement à un transducteur à des fins de vibration ; et
un circuit de détection de déviation comprenant une source d'énergie secondaire, un capteur magnétique de proximité (208) et un indicateur, la source d'énergie secondaire étant configurée pour fournir du courant électrique au capteur magnétique de proximité et à l'indicateur, dans lequel le capteur magnétique de proximité est conçu pour capter une déviation de la sonde à ultrasons et pour activer l'indicateur en réponse à celle-ci.

20. Instrument chirurgical à ultrasons comportant :
une sonde à ultrasons configurée pour conduire l'électricité, et au moins un tube, la sonde à ultrasons étant positionnée à une distance prédéterminée du, au moins un, tube et étant conçue pour être reliée fonctionnellement à un transducteur à des fins de vibration ; et
un circuit de détection de déviation comprenant une source d'énergie secondaire, un capteur d'impédance (210) et un indicateur, la source d'énergie secondaire étant configurée pour fournir du courant électrique à la sonde à ultrasons et à l'indicateur, dans lequel le capteur d'impédance est conçu pour capter un écart de l'impédance de la sonde à ultrasons par rapport à un seuil prédéterminé et pour activer l'indicateur en réponse à celui-ci.
